**Europäisches Patentamt**

**European Patent Office**

**Office européen des brevets**

(19)

(11) **EP 1 081 494 A1**

(12) **EUROPÄISCHE PATENTANMELDUNG**

(43) Veröffentlichungstag:
07.03.2001 Patentblatt 2001/10

(51) Int Cl.⁷: $G01N\ 33/22$

(21) Anmeldenummer: 99117032.5

(22) Anmeldetag: 30.08.1999

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(71) Anmelder:
- **RUHRGAS AKTIENGESELLSCHAFT**
  **45117 Essen (DE)**
- **N.V. NEDERLANDSE GASUNIE**
  **9700 MA Groningen (NL)**

(72) Erfinder:
- **Jaeschke, Manfred, Dr. Dipl.-Phys.**
  **46286 Dorsten (DE)**

- **Schley, Peter, Dipl.-Ing.**
  **45131 Essen (DE)**
- **Kleinrahm, Reiner, Dr. Dipl.-Ing.**
  **44801 Bochum (DE)**
- **Janssen-van Rosmalen, Renee, Dir. ir.**
  **9301 WB Roden (NL)**
- **Schouten, Jan A., Prof. Dr.**
  **4161 GT Monnickendam (NL)**

(74) Vertreter: **Harlacher, Mechthild, Dipl.-Ing.**
**Abt. TATP,**
**Ruhrgas AG,**
**Huttropstrasse 60**
**45138 Essen (DE)**

(54) **Verfahren zur verbrennungslosen Messung des Brennwertes von Brenngas**

(57) Die Erfindung betrifft ein Verfahren zur verbrennungslosen Messung des Brennwertes von Brenngas, insbesondere von Erdgas. Die Dielektrizitätskonstante des Erdgases wird unter Referenz - oder Betriebsbedingungen und die Dichte oder die Schallgeschwindigkeit des Brenngases unter Referenz- oder Betriebsbedingungen erfaßt. Aus den erfaßten zwei Meßsignalen wird die Gaszusammensetzung und aus dieser der volumenbezogene Brennwert abgeleitet. Auf diese Weise wird der Aufwand bei der korrelativen verbrennungslosen Messung des Brennwertes weiter verringert und ein zuverlässiges und genaues Meßverfahren zur Verfügung gestellt.

EP 1 081 494 A1

**Beschreibung**

Die Erfindung bezieht sich auf ein Verfahren zur verbrennungslosen Messung des Brennwertes von Brenngas und auf eine Verwendung des Verfahrens zur verbrennungslosen Messung und/oder Regelung der Wärmemengenzufuhr zu Gasverbrauchseinrichtungen, insbesondere zu Erdgasverbrauchseinrichtungen.

Die verbrennungslosen Verfahren zur Messung des Brennwertes bzw. zur Wärmemengenmessung haben gegenüber kalorimetrischen Verfahren, in denen eine kontrollierte Teilstrom Verbrennung des übertragenen Gasstroms durchgeführt wird, den Vorteil, daß sie wesentlich kostengünstiger sind. Allerdings ist die technische Realisierung häufig aufwendig, abgesehen davon, daß Schwierigkeiten bei der Eichung auftreten können.

Zu den verbrennungslosen Meßverfahren gehören indirekte und korrelative Verfahren. Bei den indirekten Verfahren wird die Gaszusammensetzung analysiert. Aus der Zusammensetzung des Gases kann dann mit den Brennwerten für die reinen Stoffe der Brennwert des Brenngases bestimmt werden. Diese Verfahren (z.B. die Gaschromatographie) liefern sehr genaue Ergebnisse, sind aber technisch kompliziert und daher für den Einsatz in beispielsweise Haushalten kaum geeignet. Zudem sind sie störanfällig.

Bei den korrelativen Verfahren zur Messung des Brennwertes bzw. zur Wärmemengenmessung wird ein Zusammenhang zwischen einer leicht meßbaren physikalischen oder chemischen Meßgröße und dem Brennwert ausgenutzt. Die technische Durchführung ist hierbei einfacher, jedoch werden die Reproduzierbarkeit und die Genauigkeit der Messung des Brennwertes bzw. der Wärmemenge in unerwünschtem Maße eingeschränkt.

Aufgabe der Erfindung ist es, den Aufwand bei der korrelativen verbrennungslosen Messung des Brennwertes bzw. der Messung und/oder Regelung der Wärmemengenzufuhr zu Verbrauchseinrichtungen weiter zu verringern und insbesondere ein zuverlässiges und genaues Meßverfahren zur Verfügung zu stellen.

Gelöst wird diese Aufgabe erfindungsgemäß dadurch, daß bei dem eingangs genannten Verfahren

a) die Dielektrizitätskonstante des Brenngases unter Referenz- oder Betriebsbedingungen und die Dichte oder die Schallgeschwindigkeit des Brenngases unter Referenz- oder Betriebsbedingungen erfaßt werden und

b) aus den erfaßten zwei Meßsignalen die Gaszusammensetzung abgeleitet wird und

c) aus der Gaszusammensetzung der volumenbezogene Brennwert abgeleitet wird.

Für die Messung der Dielektrizitätskonstanten, der Dichte sowie der Schallgeschwindigkeit existieren in der Praxis verschiedene bewährte Verfahren, die jeweils ohne besonderen technischen Aufwand zuverlässig und genau durchgeführt werden können. Folglich liefert die Verknüfung der Meßwerte entsprechende Ergebnisse für die Gaszusammensetzung. Die auf diese Weise bestimmte Gaszusammensetzung stimmt mit den mit Hilfe der Gaschromatographie bestimmten Anteilen von Erdgas für die Hauptkomponenten Methan, Ethan und Stickstoff besser als 0,1 % überein. Aus der Zusammensetzung wird im nächsten Schritt der volumenbezogene Brennwert bestimmt.

Besonders genaue Ergebnisse lassen sich für Brenngase erzielen, deren Brennwert bei Normbedingungen zwischen 20 und 48 MJ/m$^3$ liegt, deren auf trockene Luft bezogene relative Dichte zwischen 0,55 und 0,9 beträgt, deren Kohlenstoffdioxidanteil kleiner gleich 0,3 ist und deren Wasserstoff- und Kohlenmonoxidanteil kleiner als 0,1 bzw. 0,03 ist. Als Meßbedingungen sind Temperaturen im Bereich von 225 bis 350 K und Drücke kleiner gleich 60 MPa besonders geeignet.

Die Meßgenauigkeit der Gaszusammensetzung kann dadurch weiter erhöht werden, daß im Schritt a) zusätzlich wenigstens eine der Meßgrößen Druck, Temperatur, Kohlenstoffdioxidanteil, Kohlenstoffmonoxid- und Wasserstoffanteil des Brenngases erfaßt wird. Selbstverständlich kann die höchste Meßgenauigkeit bei der zusätzlichen Erfassuna sämtlicher zusätzlicher Meßgrößen erzielt werden.

Zum Auffinden einer geeigneten Korrelation zwischen den erfaßten Meßsignalen und der Gaszusammensetzung ist es vorteilhaft, den jeweiligen Verfahrensschritten a) und b) wenigstens einmal mehrere Meßzyklen vorzuschalten, bei denen der Schritt a) mit mehreren Referenzaasen bekannter Zusammensetzung durchgeführt wird. An dem Referenzgas werden dann die für die verschiedenen Varianten der Verfahren benötigten Meßgrößen erfaßt. In diesen Referenzzvklen wird aus dem Verhältnis der erfaßten Meßsignale eine der Zahl der Meßzyklen entsprechende Zahl von Referenzsignalmustern in Zuordnung zu den bekannten Zusammensetzungen gespeichert. Das Signalmuster aus einem späteren Meßzyklus an Brenngas unbekannter Zusammensetzung wird mit diesen Referenzsignalmustern zur Zuordnung einer bestimmten Zusammensetzung verglichen.

Zur Erhöhung der Referenzgenauigkeit sollte eine Vielzahl von Referenzzyklen durchgeführt werden, in denen die Anteile der ein-zelnen Komponenten, möglichst nacheinander, über die jeweils zu erwartenden Anteile der Komponenten am Brenngas variiert werden. Eine eindeutige und genaue Zuordnung einer bestimmten Gaszusammensetzung zu einem in einem Meßzyklus erfaßten Signalmuster eines Brenngases wird durch Interpolation der verschiedenen Referenzsignalmuster erzielt. Die Zusammensetzung der Referenzgase sollte hierbei möglichst getreu den nachfolgend zu messenden Gaszusammensetzungen gewählt werden.

Eine bevorzugte Weiterbildung der Erfindung ist dadurch gekennzeichnet, daß der jeweilige Anteil einer vorgegebenen Anzahl von Alkanen, einschließlich Methan, dadurch bestimmt wird, daß der Anteil der einzelnen Alkane, ausgenommen Methan, mit Hilfe jeweils einer zugehörigen, von einer ausgewählten physikalischen Größe, vorzugsweise dem molaren Brennwert, der Summe der vorgegebenen Alkane abhängigen Funktion bestimmt wird und daß der Methananteil aus der Differenz zwischen dem Anteil der Summe der vorgegebenen Alkane und der Summe der durch die Funktionen bestimmten Anteile der Alkane bestimmt wird.

Als vorgegebene Alkane sollten möglichst alle im Brenngas tatsächlich vorhandenen Alkane gewählt und vorgegeben werden.

Es hat sich gezeigt, daß die Anteile der Alkane bei natürlichen Brenngasen stets in einem bestimmten Verhältnis zueinander stehen, welches lediglich von einer physikalischen Meßgröße, z. B. dem molaren Brennwert, der Summe der vorgegebenen Alkane abhängt. Dies ist offenbar darauf zurückzuführen, daß Erdgase in ihrer natürlichen Form stets eine Gleichgewichtsphase durchlaufen haben, in welcher ihre - oder Flüssigkeitsphasen im Gleichgewicht zueinander standen. Jedoch ist das Verfahren nicht auf die natürlichen Brenngase, und zwar mit oder ohne Kokereigaszusatz, beschränkt. Für synthetische Gase mit Zusätzen oder für Gasgemische mit vielen Komponenten ist die Unsicherheit bei der Bestimmung der Gaszusammensetzung lediglich etwas größer.

Der molare Brennwert der Summe der vorgegebenen Alkane kann beispielsweise wiederum mit Hilfe von Referenzsignalzyklen bestimmt werden. Da bei den Referenzgasen die Zusammensetzung bekannt ist, ist auch ihr molarer Brennwert der Summe der vorgegebenen Alkane bekannt. Folglich kann aus dem Verhältnis der bei den Referenzgasen erfaßten Meßsignale eine der Zahl der Referenzmeßzyklen entsprechende Zahl von Referenzsignalmustern in Zuordnung zu den bekannten molaren Brennwerten der Summe der vorgegebenen Alkane gespeichert werden. Bei einem späteren Meßzyklus kann der molare Brennwert der Summe der vorgegebenen Alkane des Brenngases lediglich durch Vergleich der erfaßten Meßsignale mit den gespeicherten Referenzsignalmustern bestimmt werden.

Die ausgewählte physikalische Größe der Summe der vorgegebenen Alkane kann je nach Anwendungsfall beliebig gewählt werden. Z.B. kann statt mit dem molaren Brennwert mit der molaren Masse, der Dichte oder der Schallgeschwindigkeit gearbeitet werden.

Vorteilhafterweise können als Funktionen zur Bestimmung der Anteile der ein-zelnen Alkane, ausgenommen Methan, Polynome, vorzugsweise 2. Ordnung, verwendet werden.

Bei einem bevorzugten Ausführungsbeispiel wird der Methan-, Ethan-, Propan-, Isobutan-, n-Butan-, Isopentan-, n-Pentan-, Hexan-, Heptan- und Oktananteil mit Hilfe der Funktionen bestimmt. Es hat sich gezeigt, daß der Anteil aller weiteren Alkane, insbesondere bei natürlichem Brenngas, vernachlässigt werden kann. Der Zusammenhang zu dem molaren Brennwert der Summe der vorgegebenen Alkane lautet in diesem Fall z.B.:

$$X_{C2H6} = [\ \alpha_1\ (H_{CH} - H_{CH4}) + \beta_1\ (H_{CH} - H_{CH4})^2]\ x_{CH} \tag{1.1}$$

$$X_{C3H8} = [\ \alpha_1\ (H_{CH} - H_{CH4}) + \beta_2\ (H_{CH} - H_{CH4})^2]\ x_{CH} \tag{1.2}$$

$$X_{i\text{-}C4H10} = [\ \alpha_3\ (H_{CH} - H_{CH4}) + \beta_3\ (H_{CH} - H_{CH4})^2]\ x_{CH} \tag{1.3}$$

$$X_{n\text{-}C4H10} = [\ \alpha_4\ (H_{CH} - H_{CH4}) + \beta_4\ (H_{CH} - H_{CH4})^2]\ x_{CH} \tag{1.4}$$

$$X_{i\text{-}C5H12} = [\ \alpha_5\ (H_{CH} - H_{CH4}) + \beta_5\ (H_{CH} - H_{CH4})^2]\ x_{CH} \tag{1.5}$$

$$X_{n\text{-}C5H12} = [\ \alpha_6\ (H_{CH} - H_{CH4}) + \beta_6\ (H_{CH} - H_{CH4})^2]\ x_{CH} \tag{1.6}$$

$$X_{n\text{-}C6H14} = [\ \alpha_7\ (H_{CH} - H_{CH4}) + \beta_7\ (H_{CH} - H_{CH4})^2]\ x_{CH} \tag{1.7}$$

$$X_{n\text{-}C7H16} = [\ \alpha_8\ (H_{CH} - H_{CH4}) + \beta_8\ (H_{CH} - H_{CH4})^2]\ x_{CH} \tag{1.8}$$

$$X_{n-C8H18} = [\ \alpha_9\ (H_{CH} - H_{CH4}) + \beta_9\ (H_{CH} - H_{CH4})^2]\ x_{CH} \tag{1.9}$$

Hierbei sind $\alpha_i$ und $\beta_i$ Konstanten und $H_{CH4}$ der molaren Brennwert von Methan. Die Variable $H_{CH}$ steht für den molaren Brennwert der Summe der vorgegebenen Alkane ($H_{CH} = \Sigma\ X_{CH,\ i}\ H_{CH,i}$). Der Methananteil wird in diesem Fall wie folgt bestimmt:

$$X_{CH4} = X_{CH} - (X_{C2H6} + X_{C3H8} + X_{i-C4H10} + X_{n-C4H10} +$$

$$X_{i-C5H12} + X_{n-C5H12} + X_{n-C6H14} + X_{n-C7H16} + X_{n-C8H18}) \tag{2}$$

Eine Weiterbildung der Erfindung ist dadurch gekennzeichnet, daß den Verfahrensschritten a) und b) mehrere Meßzyklen vorgeschaltet werden, bei denen der Schritt a) mit mehreren Referenzgasen durchgeführt wird, deren Zusammensetzung und deren ausgewählte physikalische Meßgröße der Summe der vorgegebenen Alkane bekannt ist, daß aus den bei den Referenzgasen erfaßten Meßsignalen die Konstanten, z.B. Koeffizienten, der den Anteil der Alkane, ausgenommen Methan, beschreibenden Funktionen bestimmt werden, daß die Konstanten der Funktionen in Zuordnung zu den jeweiligen Alkanen gespeichert werden und daß der Anteil der Alkane, ausgenommen Methan, aus einem späteren Meßzyklus an Brenngas unbekannter Zusammensetzung mit Hilfe der Funktionen bestimmt wird.

Auf diese Weise können die Konstanten $\alpha_i$ und $\beta_i$ mit Hilfe bereits von zwei Referenzzyklen für alle natürlichen Erdgase aufgefunden werden. Zur Erhöhung der Meßgenauigkeit, können beliebig viele Referenzzyklen durchgeführt werden. Der Aufwand bleibt auch bei einer großen Anzahl von Referenzzyklen verhältnismäßig gering, da die Konstanten $\alpha_i$ und $\beta_i$ nur einmal bestimmt werden müssen.

Die Stoffmengenanteile der Summe der vorgegebenen Alkane lassen sich aus den molaren Anteilen von Stickstoff und Kohlenstoffioxid bestimmen:

$$X_{CH} = 1 - X_{N2} - X_{CO2} \tag{4}$$

Hierbei geben $X_{N2}$ und $X_{CO2}$ den Anteil von Stickstoff bzw. Kohlenstoffdioxid an.

Zur Erhöhung der Meßgenauigkeit kann alternativ angenommen werden, daß das Brenngas außerdem Wasserstoff und/oder Kohlenstoffmonoxid enthält. Werden sowohl der Wasserstoff als auch der Kohlenstoffmonoxidanteil berücksichtigt, so lautet die Gleichung (4):

$$X_{CH} = 1 - X_{N2} - X_{CO2} - X_{H2} - X_{CO} \tag{5}$$

Eine vorteilhafte Weiterbildung der Erfindung ist dadurch gekennzeichnet, daß aus der Zusammensetzung des Brenngases ein Wert für dessen Dielektrizitätskonstante abgeleitet wird, daß die Differnez zwischen dem abgeleiteten und dem erfaßten Wert der Dielektionskonstanten gebildet wird, daß falls die Differenz einen vorgesehenen Schwellwert überschreitet, der Anteil wenigstens einer der zu bestimmenden Komponenten des Brenngases abgeändert festgesetzt wird, daß auf der Basis des abgeänderten Wertes erneut die Zusammensetzung, die Dielektrizitätskonstante und die Differenz bestimmt werden und daß die letzten beiden Schritte solange wiederholt werden, bis die Differenz unterhalb des Schwellwertes liegt.

Der Realgasfaktor kann aus der Gaszusammensetzung abgeleitet und die molare Masse des Brenngases aus der Dichte und dem Realgasfaktor abgeleitet werden.

Der Realgasfaktor ($Z_n$) kann beispielsweise mit der im Stand der Technik bekannten AGA8-DC92-Zustandsgleichung, mit der DIN 51857 oder der ISO 6976 bestimmt werden:

$Z_n = f(T_n, P_n, X_i)$

Die Dichte unter Normbedingungen kann daraus wie folgt bestimmt werden:

$$Pn = \frac{P_n M_{mix}}{Z_n R_m T_n}$$

Eine Weiterbildung der Erfindung ist dadurch gekennzeichnet, daß aus der Zusammensetzung des Brenngases ein Wert für dessen Dichte abgeleitet wird, daß die Differenz zwischen dem abgeleiteten und dem erfaßten Wert der Dichte abgebildet wird, daß falls die Differenz einen vorgegebenen Schwellwert überschreitet, der Anteil wenigstens einer der zu bestimmenden Komponenten des Brenngases abgeändert festgesetzt wird, daß auf der Basis des abgeänderten Wertes erneut die Zusammensetzung, die Dichte und die Differenz bestimmt werden und daß die letzten beiden Schritte solange wiederholt werden, bis die Differenz unterhalb des Schwellwertes liegt.

Es hat sich gezeigt, daß auf diese Weise die Gaszusammensetzung besonders schnell bestimmt werden kann. Sollen alle oben angegebenen Gleichungen (1.1 bis 7) berücksichtigt werden, so enthalten diese bis zu 16 Unbekannte, nämlich $X_{CH4}$, $X_{N2}$, $X_{C2H6}$, $X_{C3H8}$,... $X_{n-C8H18}$, $M_{mix}$, , $X_{CH}$, $H_{CH}$, $M_{CH}$, $Z_n$. Zur Lösung dieser 16 Gleichungen kann ein Wert, z.B. die Dichte, abgeleitet und mit dem zugehörigen erfaßten Wert verglichen werden.

Das erfindungsgemäße Verfahren kann zur Bestimmung Enthalpie, der Methanzahl oder des Wobbe-Indizes des Brenngases verwendet werden.

Auch die Methanzahl kann mit dem erfindungsgemäßen Verfahren bestimmt werden. Werden als Eingangsmeßsignale für das erfindungsgemäße Verfahren diejenigen Gasbeschaffenheitsdaten verwendet, die ursprünglich für Abrechnungszwecke gemessen wurden, so kann die Methanzahl im wesentlichen mit der gleichen Genauigkeit wie bei Einsatz eines Gaschromatographen bestimmt werden. Die Abweichung der Methanzahlen beträgt weniger als 2%.

Alternativ können die Gasbeschaffenheitsdaten wie z.B. die Dichte im Normzustand aus einer Netzsimulation bestimmt werden. Netzsimulationen können derzeit die Dichte im Normzustand mit einer Unsicherheit von 1,5 Prozent genau bestimmen. Der Kohlenstoffdioxidanteil kann in diesem Fall einfach als mittlerer Wert von 1 mol % festgesetzt werden. Die auf diese Weise nach dem erfindungsgemäßen Verfahren bestimmte Methanzahl stimmt mit der bei Verwendung eines Gaschromatographen ermittelten Methanzahl im allgemeinen noch besser als zwei Prozent überein. Diese Genauigkeit genügt für die meisten Anwendungsbereiche.

Bei Anwendungen, bei denen ein Volumen- oder Massenstromzähler vorhanden ist, ist es vorteilhaft, das Meßsignal für die Schallgeschwindigkeit bei diesem Volumen- oder Massenstromzähler abzugreifen.

Bei Verwendung der für Abrechnungszwecke gemessenen Gasbeschaffenheitsdaten oder der Netzsimulationen gelingt es, ohne zusätzliche Messungen Brenngasabnehmer jederzeit über aktuelle und ggf. zukünftige Schwankungen der Methan-Zahl zu informieren. Das Gastransportnetz kann außerdem ohne Mehraufwand flexibler gesteuert werden.

Weitere vorteilhafte Ausführungsformen der Erfindung sind in den Unteransprüchen gekennzeichnet.

Im folgenden wird die Erfindung anhand eines bevorzugten Ausführungsbeispiels mit Hilfe der beigefügten Zeichnung näher erläutert.

In der Zeichnung zeigt:

Fig. 1    ein Meßdiagramm, in dem der Stoffmengenanteil von Ethan und Propan für verschiedene Gase gegen den molaren Brennwert der Summe der Kohlenwasserstoffe aufgetragen ist, und

Fig. 2    ein Ablaufdiagramm zur Bestimmung des Brennwertes gemäß einem Ausführungsbeispiel des erfindungsgemäßen Verfahrens.

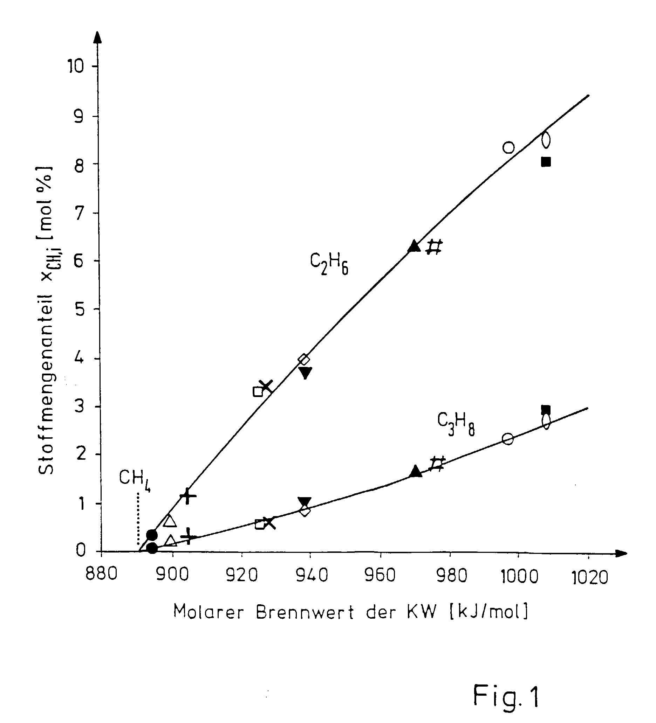

In Fig. 1 ist der molare Brennwert der Summe der Alkane ($H_{CH}$) auf der x-Achse und der Stoffmengenanteil von Ethan ($C_2H_6$) und Propan ($C_3H_8$) auf der y-Achse aufgetragen. Für verschiedene natürliche Erdgase wurden die entsprechenden Meßwerte ermittelt und aufgetragen. Der Anteil von sowohl Ethan als auch Propan wurde durch ein Polynom 2. Ordnung angenähert. Wie die Fig. 1 zeigt, kann sowohl der Anteil von Ethan als auch von Propan überraschend gut durch ein vom molaren Brennwert der Summe der Alkane abhängiges Polynom 2. Ordnung angenähert werden. Entsprechendes gilt für die weiteren Alkane bis zum Octan. Die Meßwerte für diese Alkane sind aus Gründen der Übersichtlichkeit in Fig. 1 nicht dargestellt.

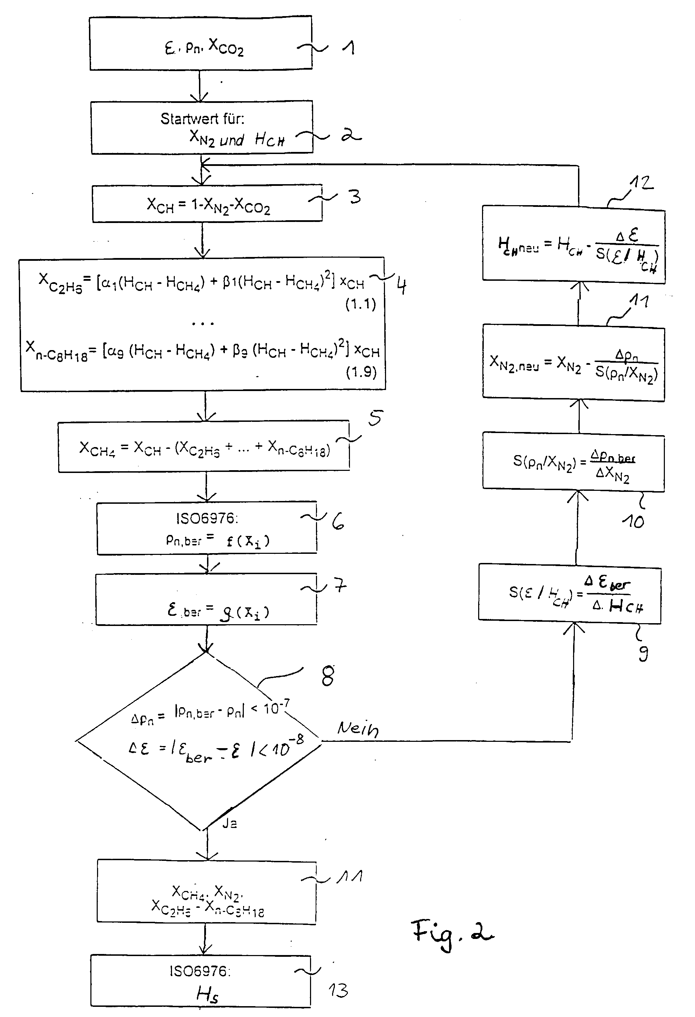

Fig. 2 zeigt ein Ablaufdiagramm zur Bestimmung des Brennwertes gemäß, einem bevorzugten Ausführungsbeispiel. Im Schritt 1 werden die Dielektrizitätskonstante des Brenngases $\varepsilon$, die Dichte unter Normbedingungen $\rho_n$ sowie der Kohlenstoffdioxidanteil $X_{CO2}$ gemessen. Der Normzustand (Index n) ist in der DIN 1343 definiert.

Im Schritt 2 wird ein Startwert für den Stickstoffanteil $X_{N2}$ und ein Startwert für den molaren Brennwert $H_{CH}$ der Summe der Alkane festgelegt. Aus dem erfaßten Kohlenstoffioxidanteil und dem Startwert für den Stickstoffanteil wird dann im Schritt 3 der Anteil der Summe der Alkane XCH bestimmt.

Im Schritt 4 wird dann mit Hilfe des anfänglichen molaren Brennwertes $H_{CH}$ Summe der Alkane der Anteil der Alkane, ausgenommen Methan, mit Hilfe der Gleichungen (1.1) bis (1.9) bestimmt.

Im Schritt 5 wird der Methananteil $X_{CH4}$ gemäß Gleichung (2) bestimmt.

Im Schritt 6 wird aus der berechneten Zusammensetzung des Gases die Dichte bei Normbedingungen $\rho_{n, ber}$ berechnet. Dies geschieht mit Hilfe der bekannten ISO 6976, und zwar über den Realgasfaktor gemäß den Gleichungen (6) und (7).

Im Schritt 7 wird aus der berechneten Zusammensetzung $X_i$ Gases die Dielektrizitätskonstante $\varepsilon_{ber}$ berechnet, und zwar mittels eines Kalibrierverfahrens.

Im Schritt 8 wird festgestellt, ob der Betrag der Differenz zwischen berechneter Normdichte $\rho_{n,ber}$ und im Schritt 1 erfaßter Normdichte kleiner als der auf 10-[7] festgelegte Schwellwert ist. Außerdem wird festgestellt, ob der Betrag der Differenz zwischen der berechneten Dielektrizitätskonstanten $\varepsilon_{ber}$ und der im Schritt 1 erfaßten Dielektrizitätskonstanten kleiner als der auf 10-[8] festgelegten Schwellwert ist. Falls nein, wird das Verfahren mit dem Schritt 9 fortgesetzt.

Im Schritt 9 wird die Empfindlichkeit bzw. Sensitivity $S(\varepsilon/H_{CH})$ bestimmt. Dazu wird ein $\Delta H_{CH}$ festgesetzt und für einen entsprechend variierten Wert die Dielektrizitätskonstante mit der Hilfe der Schritte 3 - 8 ein zweiter Wert für $\varepsilon_{ber}$ bestimmt. Die Differenz dieser beiden berechneten Werte der Dielektrizitätskonstante $\varepsilon_{ber}$ wird dann geteilt durch $\Delta H_{CH}$.

Im Schritt 10 wird die Empfindlichkeit bzw. Sensitivity $S(\rho/X_{N2})$ bestimmt. Dazu wird ein $\Delta X_{N2}$ festgesetzt, z. B. 0,01 %, und für einen entsprechend variierten Wert des Stickstoffanteils mit der Hilfe der Schritte 3 - 6 und 8 ein zweiter Wert für $\rho_{n,ber}$ bestimmt. Die Differenz dieser beiden berechneten Werte der Dichte, $\Delta_{\rho n,ber}$, wird dann geteilt durch $\Delta X_{N2}$.

Im Schritt 11 wird dann ein neuer Wert für den Stickstoffanteil $X_{N2}$, neu festgesetzt, und zwar indem von dem Startwert des Stickstoffanteils der Quotient aus dem im Schritt 8 ermittelten $\Delta_{\rho n}$ und $S(\rho_n/X_{N2})$ abgezogen wird. Mit dem neu festgesetzten Wert für den Stickstoffanteil werden die Schritte 3 bis 6 und 8 dann wiederholt. Falls im Schritt 8 erneut der Grenzwert von $\Delta\rho_n$ überschritten wird, werden erneut die Schritte 10, 11 und 3 bis 6 und 8 durchgeführt. Erst wenn im Schritt 8 der Grenzwert von 10-[7] von $\Delta\rho_n$ eingehalten wird, stehen die Anteile der Alkane sowie der Stickstoffanteil mit der gewünschten Genauigkeit fest.

Der Startwert für den Stickstoffanteil kann im Schnitt 2 z. B. dadurch bestimmt werden, daß man als molaren Brennwert der Summe der Alkane den molaren Brennwert von Methan verwendet ($H_{CH} = H_{CH4}$). Setzt man diesen Brennwert von Methan in die im Schritt 4 angegebene Gleichung ein, so erhält man nach geeigneter Auflösung einen Startwert für den Stickstoffanteil.

Im Schnitt 12 wird ein neuer Wert für den molaren Brennwert $H_{CH, neu}$ der Summe der Alkane festgelegt, und zwar indem der Quotient aus dem im Schnitt 8 ermittelten $\Delta\varepsilon$ und $S(\varepsilon/H_{CH})$ laut Schnitt 9 gebildet wird.

Mit dem neu festgesetzten Wert für den molaren Brennwert werden die Schritte 3 bis 5, 7 und 8 dann wiederholt. Falls im Schritt 8 erneut der Grenzwert von $\Delta\varepsilon$ überschritten wird, werden erneut die Schritte 9, 12 und 3 bis 5, 7 und 8 durchgeführt. Erst wenn im Schritt 8 der Grenzwert von 10-[8] von $\Delta\varepsilon$ eingehalten wird, stehen die Anteile der Alkane sowie der molare Brennwert mit der gewünschten Genauigkeit fest.

Im Schritt 13 wird schließlich aus der Zusammensetzung nach der ISO 6976 der Brennwert ermittelt.

Im Rahmen des Erfindungsgedankens sind zahlreiche Variationsmöglichkeiten gegeben. Die zur Bestimmung der Gaszusammensetzung benötigten Meßgrößen können entweder gemessen werden oder vorzugsweise auf Meßwerten beruhenden Meßsimulationen entnommen werden. Statt der absoluten Dichten kann auch die auf trockene Luft bezogene relative Dichte erfaßt werden. Die zu bestimmenden, vorgegebenen Alkane können jeweils für einen speziellen Anwendungsfall beliebig gewählt werden. Schließlich können die angegebenen Gleichungen auf jede beliebige Weise und in beliebiger Reihenfolge aufgelöst werden.

**Patentansprüche**

**1.** Verfahren zur verbrennungslosen Messung des Brennwertes von Brenngas, insbesondere von Erdgas, wobei

a) die Dielektrizitätskonstante des Brenngases unter Referenz- oder Betriebsbedingungen und die Dichte oder die Schallgeschwindigkeit des Brenngases unter Referenz- oder Betriebsbedingungen erfaßt werden und

b) aus den erfaßten zwei Meßsignalen die Gaszusammensetzung abgeleitet wird

c) und aus der Gaszusammensetzung der vorlumenbezogene Brennwert abgeleitet wird.

**2.** Verfahren nach Anspruch 1,
dadurch gekennzeichnet
daß als Referenzbedingungen für die Messung der Dielektrizitätskonstanten und/oder der Dichte und/oder der Schallgeschwindigkeit Normbedingungen eingestellt werden.

**3.** Verfahren nach Anspruch 1 oder 2,
dadurch gekennzeichnet,
daß im Schritt a) zusätzlich wenigstens eine der Meßgrößen Druck, Temperatur, Kohlenstoffdioxidanteil, Kohlenstoffmonoxid- und Wasserstoffanteil des Brenngases erfaßt wird.

**4.** Verfahren nach einem der Ansprüche 1 bis 3,
dadurch gekennzeichnet,
daß den Verfahrensschritten a) und b) mehrere Meßzyklen vorgeschaltet werden, bei denen der Schritt a) in mehreren Referenzgasen bekannter Zusammensetzung durchgeführt wird; daß aus dem Verhältnis der bei den Referenzgasen erfaßten Meßsignale eine der Zahl der Meßzyklen entsprechende Zahl von Referenzsignalmustern in Zuordnung zu den bekannten Zusammensetzungen gespeichert wird; und daß das Signalmuster aus einem späteren Meßzyklus an Brenngas unbekannter Zusammensetzung mit den Referenzsignalmustern zur Zuordnung einer bestimmten Zusammensetzung verglichen wird.

**5.** Verfahren nach einem der Ansprüche 1 bis 4,
dadurch gekennzeichnet,

daß der jeweilige Anteil einer vorgegebenen Anzahl von Alkanen, einschließlich Methan, dadurch bestimmt wird,
daß der Anteil der einzelnen Alkane, ausgenommen Methan, mit Hilfe jeweils einer zugehörigen, von einer ausgewählten physikalischen Größe, vorzugsweise dem molaren Brennwert der Summe der vorgegebenen Alkane, abhängigen Funktion bestimmt wird und
daß der Methananteil aus der Differenz zwischen dem Anteil der Summe der vorgegebenen Alkane und der Summe der durch die Funktionen bestimmten Anteile der Alkane bestimmt wird.

**6.** Verfahren nach Anspruch 5,
dadurch gekennzeichnet,
daß als Funktion ein Polynom, vorzugsweise 2. Ordnung, verwendet wird.

**7.** Verfahren nach Anspruch 5 oder 6,
dadurch gekennzeichnet,
daß der Methan-, Ethan-, Propan-, Isobutan-, n-Butan-, Isopentan-, n-Pentan-, Hexan-, Heptan- und Oktananteil mit Hilfe der Funktionen bestimmt wird.

**8.** Verfahren nach einem der Ansprüche 5 bis 7,
dadurch gekennzeichnet,

daß den Verfahrensschritten a) und b) mehrere Meßzyklen vorgeschaltet werden, bei denen der Schritt a) mit mehreren Referenzgasen durchgeführt wird, deren Zusammensetzung und deren ausgewählte physikalische Größe der Summe der vorgegebenen Alkane bekannt ist,
daß aus den bei den Referenzgasen erfaßten Meßsignalen die Konstanten, z. B. Koeffizienten, der den Anteil der Alkane, ausgenommen Methan, beschreibenden Funktionen bestimmt werden,
daß die Konstanten der Funktionen in Zuordnung zu den jeweiligen Alkanen gespeichert werden und
daß der Anteil der Alkane, ausgenommen Methan, aus einem späteren Meßzyklus an Brenngas unbekannter Zusammensetzung mit Hilfe der Funktionen bestimmt wird.

**9.** Verfahren nach einem der Ansprüche 1 bis 8,
dadurch gekennzeichnet,
daß unter der Annahme, daß das Brenngas nur aus einer vorgegebenen Anzahl von Alkanen, aus Stickstoff und Kohlenstoffioxid besteht, sowohl der Stickstoffanteil als auch die molaren Anteile der Summe der vorgegebenen Alkane iterativ bestimmt werden.

**10.** Verfahren nach einem der Ansprüche 1 bis 8,
dadurch gekennzeichnet,
daß unter der Annahme, daß das Brenngas nur aus einer vorgegebenen Anzahl von Alkanen, aus Stickstoff, Kohlenstoffdioxid sowie Wasserstoff und/oder Kohlenstoffmonoxid besteht, sowohl der Stickstoffanteil als auch die molaren Anteile der Summe der vorgegebenen Alkane iterativ bestimmt werden.

**11.** Verfahren nach einem der Ansprüche 1 bis 10,
dadurch gekennzeichnet,

daß aus der Zusammensetzung des Brenngases ein Wert für dessen Dielektrizitätskonstante abgeleitet wird,
daß die Differenz zwischen dem abgeleiteten und dem erfaßten Wert der Dilektrizitätskonstante gebildet wird,

daß falls die Differenz einen vorgesehenen Schwellwert überschreitet, der Anteil wenigstens einer der zu bestimmenden Komponenten des Brenngases abgeändert festgesetzt wird,

daß auf der Basis des abgeänderten Wertes erneut die Zusammensetzung, die Dilektrizitätskonstante und die Differenz bestimmt werden und

daß die letzten beiden Schritte solange wiederholt werden, bis die Differenz unterhalb des Schwellwertes liegt.

12. Verfahren nach einem der Ansprüche 1 bis 11,
dadurch gekennzeichnet,

daß aus der Zusammensetzung des Brenngases ein Wert für dessen Dichte abgeleitet wird,

daß die Differenz zwischen dem abgeleiteten und dem erfaßten Wert der Dichte gebildet wird,

daß falls die Differenz einen vorgesehenen Schwellwert überschreitet, der Anteil wenigstens einer der zu bestimmenden Komponenten des Brenngases abgeändert festgesetzt wird,

daß auf der Basis des abgeänderten Wertes erneut die Zusammensetzung,d ie Dichte und die Differenz bestimmt werden und

daß die letzten beiden Schritte solange wiederholt werden, bis die Differenz unterhalb des Schwellwertes liegt.

13. Verfahren nach einem der Ansprüche 1 bis 12,
dadurch gekennzeichnet,

daß die Dielektrizitätskonstante und die Dichte oder die Schallgeschwindigkeit unter Referenzbedingungen in einer gemeinsamen Meßumgebung erfaßt werden.

14. Verfahren nach einem der Ansprüche 1 bis 13,
dadurch gekennzeichnet,

daß als Referenzbedingungen für die Messung der Dielektrizitätskosntanten ein Referenzdruck von wenigstens 1 Mpa eingestellt wird.

15. Verfahren nach einem der Ansprüche 1 bis 14,
dadurch gekennzeichnet,

daß das Meßsignal für die Schallgeschwindigketi von einem Volumen- oder Massenstromzähler abgegriffen wird.

16. Verwendung des Verfahrens nach einem der Ansprüche 1 bis 15 zur Bestimmung der Enthalpie, der Methanzahl oder des Wobbe-Indizes des Brenngases.

17. Verwendung des Verfahrens nach einem der Ansprüche 1 bis 16 zur verbrennungslosen Messung und/oder Regelung der Wärmemengenzufuhr zu Gasverbrauchseinrichtungen, insbesondere zu Erdgasverbrauchseinrichtungen, wobei im Schritt a) zusätzlich das Brenngas bzw. ein Teilstrom des Brenngases durch einen Volumen- oder Massenzähler geleitet und das Volumen bzw. die Masse des zugeführten Brenngases gemessen wird.

Fig. 1

Fig. 2

**Europäisches Patentamt**

**EUROPÄISCHER RECHERCHENBERICHT**

Nummer der Anmeldung

EP 99 11 7032

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | DE 197 36 528 A (RUHRGAS AG ;NEDERLANDSE GASUNIE NV (NL))<br>25. Februar 1999 (1999-02-25)<br>* Anspruch 1 *<br>--- | 1,16 | G01N33/22 |
| A | LUEPTOW R M ET AL: "ACOUSTIC SENSOR FOR DETERMINING COMBUSTION PROPERTIES OF NATURAL GAS"<br>MEASUREMENT SCIENCE AND TECHNOLOGY,GB,IOP PUBLISHING, BRISTOL,<br>Bd. 5, Nr. 11,<br>1. November 1994 (1994-11-01), Seiten 1375-1381, XP000483676<br> ISSN: 0957-0233<br>* Zusammenfassung *<br>----- | 1 | |

| | RECHERCHIERTE SACHGEBIETE (Int.Cl.7) |
|---|---|
| | G01N |

Der vorliegende Recherchenbericht wurde für alle Patentansprüche erstellt

| Recherchenort | Abschlußdatum der Recherche | Prüfer |
|---|---|---|
| DEN HAAG | 25. Februar 2000 | Duchatellier, M |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

.....................................................................
& : Mitglied der gleichen Patentfamilie,übereinstimmendes Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**                    EP 99 11 7032

In diesem Anhang sind die Mitglieder der Patentfamilien der im obengenannten europäischen Recherchenbericht angeführten
Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

25-02-2000

| Im Recherchenbericht angeführtes Patentdokument | Datum der Veröffentlichung | Mitglied(er) der Patentfamilie | Datum der Veröffentlichung |
|---|---|---|---|
| DE 19736528     A | 25-02-1999 | WO    9910740 A | 04-03-1999 |

EPO FORM P0461

Für nähere Einzelheiten zu diesem Anhang : siehe Amtsblatt des Europäischen Patentamts, Nr.12/82

12